# EUROPEAN PATENT APPLICATION

(11) **EP 4 245 843 A1**
(43) Date of publication of application: **20.09.2023**
(21) Application number: 21891841.5
(22) Date of filing: 09.11.2021
(51) Int. Cl.: C12N 1/21, C12N 1/15, C12N 1/19, C12N 5/10, C12N 15/52, C12N 15/53, C12P 23/00

(54) **TRANSFORMANT AND METHOD FOR PRODUCING CAROTENOID COMPOSITION USING SAME**

(30) Priority: 12.11.2020 JP 2020188462
(71) Applicant: Ezaki Glico Co., Ltd., Osaka-shi Osaka 555-8502 (JP)
(72) Inventor: KUBO, Akiko, Osaka-shi, Osaka 555-8502 (JP); OHDAN, Kohji, Osaka-shi, Osaka 555-8502 (JP); TERADA, Yoshinobu, Osaka-shi, Osaka 555-8502 (JP); YAOI, Katsuro, Tsukuba-shi, Ibaraki 305-8560 (JP); FURUBAYASHI, Maiko, Sapporo-shi, Hokkaido 062-8517 (JP); MISAWA, Norihiko, Nonoichi-shi, Ishikawa 921-8836 (JP); KOBAYASHI, Miho, Nonoichi-shi, Ishikawa 921-8836 (JP); HATTAN, Junichiro, Nonoichi-shi, Ishikawa 921-8836 (JP); MAOKA, Takashi, Kouka-shi, Shiga 520-3312 (JP)
(74) Representative: Uexküll & Stolberg
(86) International application number: PCT/JP2021/041098
(87) International publication number: WO 2022/102595

(57) **Abstract**

The present invention provides a technique for synthesizing a carotenoid composition by a genetic recombination technique. A transformant in which a first promoter, an upstream gene of a carotenoid biosynthesis gene including a crtY and crtZ operably linked to the first promoter, a second promoter having higher promoter intensity than the first promoter, and a ZEP gene and a CCS gene operably linked to the second promoter have been introduced into a host cell.

## Description

### TECHNICAL FIELD

The present invention relates to a transformant and a method for producing a carotenoid composition using the same.

### BACKGROUND ART

Carotenoids are natural pigments produced by plants, algae, bacteria, and the like, and are an important group of compounds that control various physiological functions in organisms. Many carotenoids have been isolated so far, and the health function of carotenoids, which are present only in trace amounts in nature, has begun to attract attention, however, since the carotenoids are in trace amounts, commercial production thereof is expensive and difficult.

For this reason, in recent years, biotechnology research for inexpensively producing carotenoids with microorganisms has been actively conducted. Carotenoid biosynthetic pathways and biosynthesis genes in plants have been largely revealed by 2000. Many attempts have been made to identify genes involved in biosynthesis of carotenoids from upstream, prepare transformants in which the identified genes are introduced into microorganisms, and produce carotenoids by the transformants.

The microorganism that has been most studied as a host for carotenoid production is E. coli. E. coli synthesizes isopentenyl diphosphate (IPP) and dimethylallyl diphosphate (DMAPP), which is an isomer thereof, through a nonmevalonate pathway (MEP pathway) as a basic metabolic pathway of isoprenoids including carotenoids (Non Patent Document 1). The synthesized IPP and DMAPP are metabolized to geranyl diphosphate (GPP) and farnesyl diphosphate (FPP) by a continuous condensation reaction via a prenyl group transferase.

Escherichia coli is a carotenoid non-producing bacterium, and does not possess an enzyme gene necessary for carotenoid synthesis after FPP, however, it becomes possible to synthesize various carotenoids by incorporating therein carotenoid biosynthesis genes such as a geranylgeranyl pyrophosphate (GGPP) synthase gene (crtE), a phytoene synthase gene (crtB), a phytoene desaturase gene (crtI) that synthesizes lycopene, a lycopene cyclase gene (crtY) that synthesizes β-carotene, and a β-carotene hydroxylase gene (crtZ) that synthesizes β-cryptoxanthin and zeaxanthin from the upstream depending on the carotenoid intended to be produced.

On the other hand, even if the carotenoid is found to be producible in this way, the actual production amount is often far from the practical production level simply by introducing a biosynthesis gene group of the carotenoid. This is attributed to the fact that E. coli can produce only a small amount of FPP as a precursor of carotenoid synthesis. For this reason, for mass production of carotenoids, pathway engineering for the purpose of increasing FPP production has been studied.

The approach of the pathway engineering is roughly divided into a method of modifying an endogenous metabolic pathway including the MEP pathway and a method of introducing a mevalonate (MVA) pathway gene group derived from a heterologous organism.

As a representative example of the former approach, there is a method of remarkably increasing the carotenoid production amount with E. coli in which a type 1 IPP isomerase gene (idi) derived from S. cerevisiae or Haematococcus pluvialis is highly expressed (Non-Patent Document 2), and for example, it has been reported that E. coli in which idi and, crtE, crtB, crtI, crtY, and crtZ derived from Pantoea ananatis are introduced can predominantly produce zeaxanthin (Non-Patent Document 3).

As a representative example of the latter approach, it has been found that deuterated zeaxanthin is synthesized using deuterated D-MVA lactone as a substrate by utilizing E. coli in which an MVA pathway gene group (including a type 2 IPP isomerase gene) derived from an actinomycete Streptomyces sp. CL190 strain is functionally expressed (Non-Patent Document 4). This approach is believed to be a powerful and effective means in pathway engineering studies for E. coli-hosted carotenoid production (Non-Patent Document 5).

On the other hand, it has been found that a zeaxanthin epoxidase (ZEP) gene synthesizes antheraxanthin from zeaxanthin, and a capsanthin-capsorubin synthase (CCS) gene synthesizes capsanthin from antheraxanthin, respectively, (Non Patent Document 6). However, there is no report that capsanthin was synthesized by introducing these genes into a host together with other carotenoid biosynthesis genes although it has been a long time since these genes were found.

### PRIOR ART DOCUMENTS

### NON-PATENT DOCUMENTS

Non-Patent Document 1: Rohmer, M. et al.: Biochem. J., 295, 517 (1993)
Non-Patent Document 2: Kajiwara, S. et al.: Biochem. J., 324, 421 (1997)
Non-Patent Document 3: Takemura, M. et al.: Tetrahedron Letters 56, 6063 (2015).
Non-Patent Document 4: Kakinuma, K. et al.: J. Am. Chem. Soc., 123, 1238 (2001).
Non-Patent Document 5: Journal of the Society for Biotechnology, Japan, Vol. 93, No. 7, 397 (2015).
Non-Patent Document 6: Bouvier, F. et al. The Plant Journal, 6 (1), 45 (1994)

### SUMMARY OF THE INVENTION

### PROBLEMS TO BE SOLVED BY THE INVENTION

Unlike lycopene, carotene, cryptoxanthin, and zeaxanthin, capsanthin cannot be synthesized by a method using a known recombinant microorganism. In fact, when the present inventors introduced CrtE gene, CrtB gene, CrtI gene, CrtY gene, CrtZ gene, ZEP gene, and CCS gene together with IDI gene into E. coli, capsanthin was not produced at all.

An object of the present invention is to provide a technique for producing a composition containing a carotenoid that has not been able to be synthesized so far, such as capsanthin, by a genetic recombination technique.

### MEANS FOR SOLVING THE PROBLEM

The present inventor has conceived a novel idea of controlling the expression levels of only some genes in the biosynthesis gene group of capsanthin to be relatively high. Therefore, in order to obtain a relatively large amount of capsanthin substrate, a recombinant vector was constructed so that the expression level of the upstream gene in the biosynthesis gene group of capsanthin was relatively large, and this was transformed to try to synthesize capsanthin. However, in this method, incorporation of idi did not produce capsanthin at all.

Therefore, when the idea was changed so as to construct a recombinant vector so that the expression level of a downstream gene in the biosynthesis gene group of capsanthin becomes relatively high, it was unexpectedly found that the transformant can produce capsanthin. Furthermore, it was also unexpectedly found that when the ZEP gene and the CCS gene are incorporated into a recombinant vector such that the ZEP and the CCS are expressed so as to be indirectly bound via a multimer of a predetermined affinity protein, other various carotenoids such as capsorubin, cucurbitaxanthin A, and the like can also be produced. The present invention has been completed by further conducting studies based on this finding.

That is, the present invention provides inventions of the following aspects.
Item 1. A transformant in which a first promoter, and an upstream gene of a carotenoid biosynthesis gene including crtY and crtZ operably linked to the first promoter, and a second promoter having a higher promoter intensity than that the first promoter, and a ZEP gene and a CCS gene operably linked to the second promoter have been introduced into a host cell.
Item 2. The transformant according to item 1, wherein a difference in promoter intensity between the first promoter and the second promoter is equal to or greater than a difference in promoter intensity between a promoter unique to a carotenoid biosynthesis gene and PBAD.
Item 3. The transformant according to item 1 or 2, wherein the upstream gene further contains crtl; crtI and crtB; or crtI, crtB and crtE.
Item 4. The transformant according to any one of items 1 to 3, wherein the first promoter is Plac or a promoter unique to a carotenoid biosynthesis gene.
Item 5. The transformant according to any one of items 1 to 4, wherein the second promoter is PBAD or Ptac.
Item 6. The transformant according to any one of items 1 to 5, wherein additionally, an S protein gene, an S tag gene, and a linker gene have been introduced into the host cell, the S-tag gene is linked to each of the ZEP gene and the CCS gene, and the linker gene is interposed and linked between the ZEP gene and the S-tag gene and/or between the CCS gene and the S-tag gene.
Item 7. The transformant according to item 6, wherein the S-tag encoded by the S-tag gene is any one of the following polypeptides (1) and (2): (1) a polypeptide containing a base sequence encoding the amino acid sequence set forth in SEQ ID NO: 4; (2) a polypeptide that has a sequence identity of 90% or more other than the 12th sequence with respect to the amino acid sequence set forth in SEQ ID NO: 4 and specifically binds to S protein.
Item 8. The transformant according to any one of items 1 to 7, wherein the linker gene is interposed and linked at least between the ZEP gene and the S-tag gene.
Item 9. The transformant according to any one of items 1 to 8, wherein the linker gene is interposed and linked between the ZEP gene and the S-tag gene, and is not interposed between the CCS gene and the S-tag gene.
Item 10. A method for producing a carotenoid composition, including a step of culturing the transformant according to any one of items 1 to 9.
Item 11. The production method according to item 10, wherein the carotenoid composition contains capsanthin.
Item 12. The production method according to item 10 or 11, wherein the carotenoid composition contains capsorubin, capsanthin 3'-acetate, capsorubin 3-acetate, cucurbitaxanthin A, capsorubin diacetate, capsanthin 3,6-epoxide, and/or capsanthin 3,6-epoxide 3'-acetate.

### ADVANTAGES OF THE INVENTION

According to the present invention, it is possible to produce a composition containing a carotenoid that has not been able to be synthesized so far, such as capsanthin, by a genetic recombination technique. The mechanism by which such an effect is obtained is not clear, but is considered as follows. For CCS, not only the activity of synthesizing capsanthin from antheraxanthin, but also the side activity of synthesizing β carotene from lycopene upstream of the carotenoid biosynthetic pathway was found. For this reason, it is considered that when the amount of the upstream substrate was increased, most of the functions of CCS were utilized as a catalyst for a reaction for synthesizing β carotene from lycopene, and the CCS did not function to exhibit the activity of synthesizing capsanthin. It is considered that the expression levels of zeaxanthin and the gene upstream thereof are relatively lowered, and the expression levels of the ZEP gene and the CCS gene downstream of the gene related to the synthesis of zeaxanthin are increased, so that excessive accumulation of zeaxanthin and the intermediate upstream thereof is suppressed, and CCS that has not been able to function to exhibit the activity of synthesizing capsanthin can be caused to function for the first time.

### BRIEF DESCRIPTION OF THE DRAWINGS

Fig. 1 shows a carotenoid biosynthetic pathway and a group of enzymes mediating the pathway.
Fig. 2 is a schematic diagram of a recombinant vector produced in Comparative Example 1.
Fig. 3 is a schematic diagram of recombinant vectors produced in Comparative Example 2 (Comparative Example 2-1 and Comparative Example 2-2).
Fig. 4 shows an HPLC chromatogram of carotenoid compositions obtained in Comparative Example 2 (Comparative Example 2-1 and Comparative Example 2-2).
Fig. 5 is a schematic diagram of a recombinant vector produced in Example 1.
Fig. 6 shows an HPLC chromatogram of a carotenoid composition obtained in Example 1.
Fig. 7 shows a UPLC chromatogram (a) of a carotenoid composition obtained in Example 1, an absorption spectrum (b) of capsanthin, and a mass spectrum (c) of capsanthin.
Fig. 8 is a schematic diagram of recombinant vectors produced in Example 2 (Example 2-1 and Example 2-2).
Fig. 9 shows an HPLC chromatogram of carotenoid compositions obtained in Example 2 (Example 2-1 and Example 2-2).
Fig. 10 shows the production amount of capsanthin in carotenoid compositions obtained in Example 2 (Example 2-1 and Example 2-2).
Fig. 11 is a schematic diagram of recombinant vectors produced in Example 3 (Example 3-1 to Example 3-3).
Fig. 12 schematically shows a cooperative system of CCS and ZEP using S protein constructed in an expression system according to Example 3 (Example 3-1 to Example 3-3).
Fig. 13 shows the analysis results of carotenoid compositions obtained in Example 3 (Example 3-1 to Example 3-3).
Fig. 14 shows the analysis results of a carotenoid composition obtained in Example 3 (Example 3-2).

### EMBODIMENTS OF THE INVENTION

### 1. Transformant

The transformant of the present invention is a transformant in which a first promoter, an upstream gene of a carotenoid biosynthesis gene including crtY and crtZ operably linked to the first promoter, a second promoter having a higher promoter intensity than the first promoter, and a ZEP gene and a CCS gene operably linked to the second promoter have been introduced into a host cell. This allows for the expression of a carotenoid biosynthesis enzyme in a host cell and the production of a carotenoid composition.

### 1-1. Carotenoid biosynthesis enzyme and gene encoding the same (carotenoid biosynthesis gene)

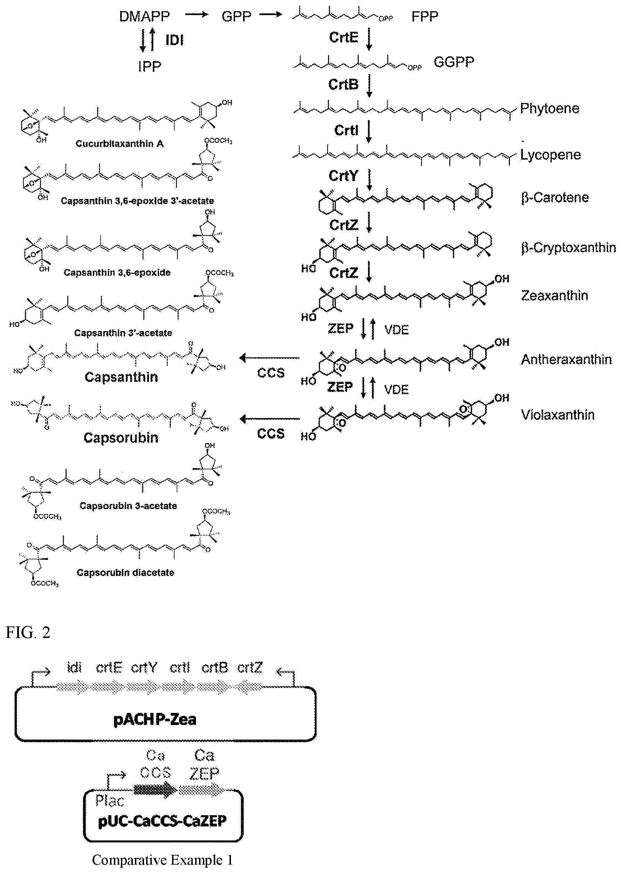

Fig. 1 shows a carotenoid biosynthetic pathway and a group of enzymes mediating the pathway. Fig. 1 shows an example in which a carotenoid is biosynthesized using recombinant E. coli.

CrtE is a GGPP synthase that synthesizes geranylgeranyl pyrophosphate (GGPP) from farnesyl diphosphate (FPP), and hereinafter, the gene encoding CrtE is referred to as "crtE".

CrtB is a phytoene synthase that synthesizes phytoene from GGPP, and hereinafter, the gene encoding CrtB is described as "crtB".

Crtl is a phytoene desaturase that synthesizes lycopene from phytoene, and hereinafter, the gene encoding CrtI is referred to as "crtI".

CrtY is a lycopene cyclase that synthesizes β carotene from lycopene, and hereinafter, the gene encoding CrtY is described as "crtY".

CrtZ is a β-carotene hydroxylase that synthesizes β-cryptoxanthin and zeaxanthin from β-carotene, and hereinafter, the gene encoding CrtZ is referred to as "crtZ".

ZEP is a zeaxanthin epoxidase that synthesizes antheraxanthin and violaxanthin from zeaxanthin.

CCS is a capsanthin/capsorubin synthase known to synthesize capsanthin and capsorubin from antheraxanthin and violaxanthin, respectively. Furthermore, according to findings found by the present inventors, CCS is also a synthase that synthesizes capsanthin 3'-acetate and cucurbitaxanthin A from antheraxanthin, and synthesizes capsorubin 3-acetate, capsorubin diacetate, capsanthin 3,6-epoxide, and capsanthin 3,6-epoxide 3'-acetate from violaxanthin.

The gene encoding the carotenoid biosynthesis enzyme is a known gene, and its specific sequence can be appropriately obtained from the official database and the like.

The organism from which these carotenoid biosynthesis genes are derived is not particularly limited, and is selected without particular limitation from organisms that produce carotenoids corresponding to these genes. Specific origin organisms may be any plant or microorganism, and specifically include Capsicum (e.g., Capsicum annuum), Lilium (e.g., Lilium lancifolium), Arabidopsis (e.g., Arabidopsis thaliana), Zea (e.g., Zea Mays), Prunus (e.g., Prunus armeniaca), Ipomoea (e.g., Ipomoea nil), Gentiana (e.g., Gentiana lutea), Marchantia (e.g., Marchantia Polymorpha), Pantoea (e.g., Pantoea ananas), Agrobacterium (e.g., Agrobacterium aurantiacum), Heamatococcus, Monoraphidium, Phaffia (Xanthophyllomyces), Paracoccus, Brevundimonas, Gordonia (Rhodococcus), Nostoc, Brevundimonas, Gloeobacter, and the like, and preferably include Capsicum, Lilium, and Pantoea. In addition, the difference of the organism from which each gene is derived is not limited, but the same organism is preferable.

The carotenoid biosynthesis gene that can be used in the present invention includes not only those consisting of a base sequence that can be obtained from the official database and a codon optimized sequence thereof, but also those consisting of a base sequence similar to (specifically, similar to the degree of similarity of the base sequences of (I-2), (II-2), (III-2), and (I-3), (II-3), and (III-3) to the base sequences of (I-1), (II-1), and (III-I) below) the sequence as long as they encode an enzyme having carotenoid biosynthesis enzyme activity.

Preferable examples of the base sequence of the carotenoid biosynthesis gene include the following.
(I-1) Base sequences of the crtE, crtY, crtI, crtB, and crtZ genes represented by DDBJ Accession No. D90087,
(II-1) A base sequence of the ZEP gene represented by DDBJ accession number XM_016705616,
(III-1) A base sequence of the CCS gene represented by DDBJ Accession No. X76165,
(I-2) A base sequence of a nucleic acid that is a gene encoding each of an enzyme having GGPP synthase activity that synthesizes geranylgeranyl pyrophosphate (GGPP) from farnesyl diphosphate (FPP), an enzyme having phytoene synthase activity that synthesizes phytoene from GGPP, an enzyme having phytoene desaturase activity that synthesizes lycopene from phytoene, an enzyme having lycopene cyclase activity that synthesizes β carotene from lycopene, and an enzyme having β carotene hydroxylase activity that synthesizes β cryptoxanthin and zeaxanthin from β carotene, and being a base sequence of a nucleic acid hybridizing under stringent conditions with a nucleic acid containing base sequences complementary to the base sequences shown in the above (I-1), respectively,
(II-2) A base sequence of a nucleic acid that is a gene encoding an enzyme having zeaxanthin epoxidase activity that synthesizes antheraxanthin and violaxanthin from zeaxanthin, and being a base sequence of a nucleic acid hybridizing under stringent conditions with a nucleic acid containing base sequences complementary to the base sequences shown in the above (II-1),
(III-2) A base sequence of a nucleic acid that is a gene encoding enzymatic activity that synthesizes capsanthin and capsorubin from antheraxanthin and violaxanthin, respectively, and synthesizes, additionally, capsorubin, capsanthin 3'-acetate, capsorubin 3-acetate, cucurbitaxanthin A, capsorubin diacetate, capsanthin 3,6-epoxide, and capsanthin 3,6-epoxide 3'-acetate, and being a base sequence of a nucleic acid hybridizing under stringent conditions with a nucleic acid containing base sequences complementary to the base sequences shown in the above (III-I),
(I-3) A base sequence that is a gene encoding each of an enzyme having GGPP synthase activity that synthesizes geranylgeranyl pyrophosphate (GGPP) from farnesyl diphosphate (FPP), an enzyme having phytoene synthase activity that synthesizes phytoene from GGPP, an enzyme having phytoene desaturase activity that synthesizes lycopene from phytoene, an enzyme having lycopene cyclase activity that synthesizes β carotene from lycopene, and an enzyme having β carotene hydroxylase activity that synthesizes β cryptoxanthin and zeaxanthin from β carotene, and having 90% or more homology with each of base sequences complementary to the base sequences shown in the above (I-1),
(II-3) A base sequence that is a gene encoding an enzyme having zeaxanthin epoxidase activity that synthesizes antheraxanthin and violaxanthin from zeaxanthin, and having 90% or more homology with the base sequences shown in the above (II-1),
(III-3) A base sequence that is a gene encoding enzymatic activity that synthesizes capsanthin and capsorubin from antheraxanthin and violaxanthin, respectively, and synthesizes, additionally, capsorubin, capsanthin 3'-acetate, capsorubin 3-acetate, cucurbitaxanthin A, capsorubin diacetate, capsanthin 3,6-epoxide, and capsanthin 3,6-epoxide 3'-acetate, and having 90% or more homology with the base sequences shown in the above (III-1).

In the above (I-2), (II-2), and (III-2), the phrase "under stringent conditions" refers to conditions for incubating for 4 hours to overnight at 50°C to 65°C in 6 × SSC (1 x SSC denotes 0.15 M NaCl, 0.015 M sodium citrate, pH 7.0) containing 0.5% SDS, 5 × Denhartz's [Denhartz's, 0.1% Bovine Serum Albumin (BSA), 0.1% Polyvinylpyrrolidone, 0.1% Ficoll 400], and 100 µg/ml salmon sperm DNA. Hybridization under stringent conditions is specifically performed by the following method. That is, a nylon membrane on which a DNA library or a cDNA library is immobilized is prepared, and the nylon membrane is blocked at 65°C in a prehybridization solution containing 6 × SSC, 0.5% SDS, 5 × Denhartz's, and 100 µg/ml salmon sperm DNA. Each probe labeled with ³²P is then added and incubated overnight at 65°C. This nylon membrane is washed in 6 × SSC for 10 minutes at room temperature, in 2 × SSC containing 0.1% SDS for 10 minutes at room temperature, in 0.2 × SSC containing 0.1% SDS at 45°C for 30 minutes, and then autoradiography is performed, and a nucleic acid specifically hybridized with the probe can be detected.

In the above (I-3), (II-3), and (III-3), the homology may be 90% or higher, preferably 95% or higher, more preferably 98% or higher, and still more preferably 98.5% or more, more preferably 99% or more, even more preferably 99.5% or more, and particularly preferably 99.9% or more. "Homology" is calculated using publicly available or commercially available software with algorithms that compare a reference sequence as a query sequence. Specifically, BLAST, FASTA, GENETYX (manufactured by GENETYX CORPORATION), and the like can be used, and these may be used by being set as default parameters.

Furthermore, in the carotenoid biosynthesis gene described above, the full-length sequence may be incorporated into a recombinant vector, or may be incorporated into a recombinant vector in a state in which an unnecessary sequence in expression of a target enzyme in a host is removed. For example, when the organism from which the carotenoid biosynthesis gene is derived is a plant and the host is a non-plant cell, it is preferable to incorporate the carotenoid biosynthesis gene into the recombinant vector in a state where the signal sequence is removed from the viewpoint of further improving the production amount of capsanthin. The signal sequence of each plant-derived gene can also be easily acquired by using the official database or various gene analysis tools.

In the carotenoid biosynthesis gene group, crtE, crtB, crtI, crtY, and crtZ are classified into "upstream genes", and the ZEP gene and the CCS gene are classified into "downstream genes", in the following.

The upstream gene of the carotenoid biosynthesis gene is operably linked to a first promoter and the downstream gene of the carotenoid biosynthesis gene is operably linked to a second promoter. That is, the first promoter is a region that performs transcription control of an upstream gene, and the second promoter is a region that performs transcription control of a downstream gene. Operably linked means that the control factor and the above gene are linked in a state where they can operate in the host cell.

### 1-2. Promoter

Regarding the relationship between the first promoter and the second promoter, the second promoter is designed to have a higher promoter intensity than the first promoter. For example, the promoter intensity of the second promoter is preferably three times or more the promoter intensity of the first promoter. Promoter intensity refers to the frequency of transfer initiation, and is measured, for example, in Pbla unit (Pbla unit; Deuschle et al., EMBO J., 5: 2987-2994 (1986)), in miller unit (Miller units; also referred to as β-galactosidase unit; 5000 times the Pbla unit (Lanzer et al., PNAS USA, 85, 8973-8977 (1988)), RPU (Relative promoter units; Meyer et al., Nature Chemical Biology 15, 196-204 (2019)), or the like.

In the present invention, the first promoter and the second promoter control the expression level of the upstream gene of the carotenoid biosynthesis gene so as to be relatively low and the expression level of the downstream gene so as to be relatively high, thereby allowing the synthesis of the carotenoid.

From the viewpoint of further improving the synthesis amount of the carotenoid, the difference in promoter intensity between the first promoter and the second promoter is preferably greater than or equal to the difference in promoter intensity between the promoter unique to the carotenoid biosynthesis gene (preferably a promoter which is naturally present in Pantoea ananatis, more preferably a promoter which is included in DDBJ accession number D90087) and the PBAD promoter, and more preferably greater than or equal to the difference in promoter intensity between the promoter unique to the carotenoid biosynthesis gene (preferably a promoter which is naturally present in Pantoea ananatis, more preferably a promoter which is included in DDBJ accession number D90087) and the tac promoter.

The first promoter and the second promoter are not particularly limited as long as they are designed so that the second promoter has a relatively higher promoter intensity, and are appropriately selected according to the type of the host into which the recombinant vector incorporating these promoters is introduced. The first promoter and the second promoter may be natural promoters or artificial promoters.

Specific examples of the promoter used as the first promoter and the second promoter include a lac promoter (Plac), a promoter unique to the carotenoid biosynthesis gene, a BAD promoter (PBAD), a tac promoter (Ptac), a trc promoter, a T7 promoter, a T7lac promoter, a T5 promoter, a UV5lac promoter, a L8-UV5lac promoter, a cspA promoter, a CAG promoter, a CMV promoter, an RSV promoter, and the like, in prokaryotes such as E. coli and the like, and include a GAL1 promoter, an NMT1 promoter, a TEF1 promoter, an ADH1 promoter, a TPI1 promoter, an HXT7 promoter, a TDH3 promoter, a PGK1 promoter, a PYK1 promoter, a CAG promoter, a CMV promoter, an RSV promoter, and the like, in yeasts.

The promoter unique to the carotenoid biosynthesis gene is a promoter that is naturally provided in the biosynthesis of carotenoid. The promoter unique to the carotenoid biosynthesis gene can be used without particular limitation, and in the organism from which the carotenoid biosynthesis gene is derived, a promoter that is naturally provided as a promoter of the carotenoid biosynthesis gene can be used. As an example, in a case where the gene placed under the control of the promoter unique to the carotenoid biosynthesis gene is derived from Pantoea ananatis, preferably a promoter naturally provided in Pantoea ananatis, more preferably a promoter included in DDBJ accession number D90087 can be used as the promoter unique to the carotenoid biosynthesis gene.

Among various promoters including these examples, two types having different promoter intensities can be selected, and the one having a lower promoter intensity can be selected as the first promoter and the one having a lower promoter intensity can be selected as the second promoter. Furthermore, in order to further improve the synthesis amount of the carotenoid, a combination of two promoters can be selected from among various promoters including the above examples so that the promoter intensities show the difference in promoter intensity described above, and the one with the lower promoter intensity can be selected as the first promoter and the one with the lower promoter intensity can be selected as the second promoter.

More preferably, examples of the first promoter include Plac and promoters unique to carotenoid biosynthesis genes, and examples of the second promoter include PBAD and Ptac, and even more preferably Ptac.

In addition, the control of the expression level by using the first promoter and the second promoter having different promoter intensities as described above is performed so as to suppress the reaction of synthesizing β carotene from lycopene by CCS. Therefore, the upstream gene expressed by the first promoter should include at least crtY and crtZ.

Other upstream genes may be placed under the control of the first promoter as with crtY and crtZ, or may be placed under the control of other promoters. Preferably, the other upstream gene is preferably placed under the control of the first promoter. A specific example of a case where another upstream gene is placed under the control of the first promoter is a case where (i) crtI is further contained; a case where (ii) crtI and crtB are contained; and a case where (iii) crtI, crtB, and crtE are contained, as the upstream gene. In addition, IPP isomerase gene (idi) may be contained together with other upstream genes, that is, (iv) crtI, crtB, crtE, and idi may be further placed under the control of the first promoter.

Both the first promoter and the gene placed under the control thereof and the second promoter and the gene placed under the control thereof may be incorporated into one expression vector to constitute one recombinant vector, or may be incorporated into separate expression vectors to constitute the first recombinant vector and the second recombinant vector, respectively.

### 1-3. Gene for expression of cooperative system of CCS and ZEP via S protein

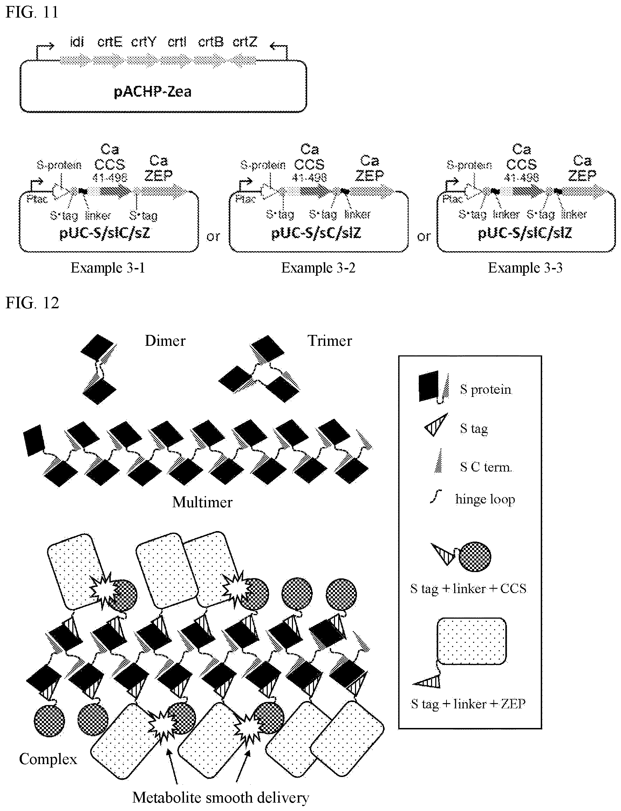

CCS also has lycopene β-cyclase activity possessed by CrtY in addition to capsanthin/capsorubin synthesis activity. Due to such dual activity of CCS, even if the CCS gene is efficiently expressed in E. coli, a reaction of receiving lycopene as a substrate from CrtI and synthesizing β-carotene by acting in cooperation with phytoene desaturase CrtI rather than ZEP may be prioritized. Therefore, in order to further increase the production amount of a reaction product by CCS by selectively reacting CCS with ZEP in a cooperative manner, it is preferable that the CCS gene and the ZEP gene are introduced into the host cell in a mode in which a cooperative system of CCS and ZEP via S protein is expressed, as follows. Fig. 12 is a diagram schematically illustrating the cooperation system.

In an embodiment of the present invention in which a cooperative system of CCS and ZEP via S protein is expressed, further, an S protein gene, an S tag gene, and a linker gene are introduced into a host cell, the S tag gene is linked to each of the ZEP gene and the CCS gene, and the linker gene is interposed and linked between the ZEP gene and the S tag gene and/or between the CCS gene and the S tag gene.

The S-tag and the S-protein are in a mutually specific relationship of affinity tag and affinity protein, and are polypeptides constituting one RNase A molecule. An N-terminal 20 amino acid fragment obtained by cleaving RNase A with subtilisin is an S tag, and a remaining fragment composed of about 100 amino acids is an S protein. The S-tag and S-protein can be non-covalently bound to reproduce the original conformation, resulting in RNase activity (Lopez-Alonso, J.P., Bruix, M., Font, J., Ribo, M., Vilanova, M., Rico, M., Gotte, G., Libonati, M., Gonzalez, C. and Laurents, D.V. (2006) J.Biol.Chem. 281, 9400-9406).

S protein and RNase A have a property that a part of these molecules swaps a C-terminal β-strand between molecules to form a multimer, such as a homo "dimer", a homo "trimer", or a trimer or more homo "multimer" as shown in Fig. 12.

On the other hand, the ZEP gene and the CCS gene to which the S-tag gene is linked are expressed as S-tag-fused ZEP and S-tag-fused CCS, respectively, in the host cell. Furthermore, since the linker gene is interposed and linked between the ZEP gene and the S-tag gene and/or between the CCS gene and the S-tag gene, either or both of the S-tag-fused ZEP and S-tag-fused CCS expressed in the host cell take the form of a fusion protein via a linker between the S-tag and the enzyme. In the schematic diagram of Fig. 1, an example is shown in which both the S-tag-fused ZEP and the S-tag-fused CCS are expressed in the form of a fusion protein via a linker between the S-tag and the enzyme (In the figure, "S tag + linker + CCS" and "S tag + linker + ZEP" are illustrated.).

Since such an S-tag-fused ZEP and S-tag-fused CCS are co-expressed with S protein, a complex in which the fused ZEP and the fused CCS are indirectly bound to the S protein multimer via the S tag is formed as shown in Fig. 12. By forming such a complex, the ZEP and the CCS physically approach each other in the host cell, so that the CCS can selectively cooperatively react with the ZEP. That is, the CCS can smoothly receive metabolites by the ZEP. This makes it possible to obtain more carotenoids produced by CCS metabolism. More specifically, it is possible to increase the amount of capsanthin produced and/or to increase the amount of capsorubin, capsanthin 3'-acetate, capsorubin 3-acetate, cucurbitaxanthin A, capsorubin diacetate, capsanthin 3,6-epoxide, and/or capsanthin 3,6-epoxide 3'-acetate produced.

As described above, S protein is known as a cleavage product of about 100 amino acids by subtilisin of RNase A, and thus its amino acid sequence is known (specific examples thereof include SEQ ID NO: 2), and the base sequence of S protein gene is also known (specific examples thereof include SEQ ID NO: 1). Examples of the base sequence of the S protein gene that can be used in the present invention include the following.
(IV-1) A base sequence represented by SEQ ID NO: 1,
(IV-2) A base sequence of a nucleic acid that is a gene encoding a protein that forms a multimer and specifically binds to an S tag, and being a base sequence of a nucleic acid hybridizing under stringent conditions with a nucleic acid containing base sequences complementary to the base sequences shown in the above (IV-1), and
(IV-3) A base sequence that is a gene encoding a protein that forms a multimer and specifically binds to an S tag, and having 90% or more homology with the base sequences shown in the above (IV-1).

The phrase "under stringent conditions" in the above (IV-2) is the same as those described for the above (I-2), (II-2), and (III-2). The "homology" in the above (IV-3) and preferred examples thereof are the same as those described in the above (I-3), (II-3), and (III-3).

As described above, the S-tag is known as a product of cleavage of 20 amino acids at the N-terminus of RNase A by subtilisin, and thus the amino acid sequence thereof is known (specific examples of the amino acid sequence include an amino acid sequence in which the 12th position of SEQ ID NO: 4 is histidine instead of phenylalanine.), and the base sequence of the S protein gene is also known (specific examples thereof include a base sequence in which positions 34 to 36 of SEQ ID NO: 3 are sequences encoding histidine instead of the sequence encoding phenylalanine.).

Examples of the S tag that can be used in the present invention include any of the following.
(1) A polypeptide containing a base sequence encoding the amino acid sequence set forth in SEQ ID NO: 4.
(2) A polypeptide that has a sequence identity other than the 12th sequence with respect to the amino acid sequence set forth in SEQ ID NO: 4 of 90% or more, preferably 95% or more, and specifically binds to S protein.

In the amino acid sequence set forth in SEQ ID NO: 4, the 12th amino acid sequence is substituted from histidine in the wild-type S-tag to phenylalanine for the purpose of suppressing the influence on RNase activity caused by binding of the S-tag to S protein in the host cell.

In the polypeptide of the above (2), the sequence identity to the amino acid sequence set forth in SEQ ID NO: 4 is a sequence identity calculated as compared with the amino acid sequence set forth in SEQ ID NO: 4. In addition, the "sequence identity" indicates a value of identity of an amino acid sequence obtained by bl2seq program (Tatiana A. Tatsusova, Thomas L. Madden, FEMS Microbiol. Lett., Vol. 174, p 247-250, 1999) of BLASTPACKAGE [sgi 32 bit edition, Version 2.0.12; available from National Center for Biotechnology Information (NCBI)]. The parameter may be set to Gap insertion Cost value: 11 and Gap extension Cost value: 1.

The base sequence of the S-tag gene that can be used in the present invention is not particularly limited as long as it is a base sequence encoding the S-tag, and preferred examples thereof include the following.
(V-1) A base sequence represented by SEQ ID NO: 3 (that is, a base sequence encoding the amino acid sequence represented by SEQ ID NO: 4),
(V-2) A base sequence that is a gene encoding a peptide that specifically binds to S protein, and having 90% or more, preferably 95% or more homology with the base sequence shown in (V-1) above (with the proviso that the base sequence other than positions 34 to 36 of SEQ ID NO: 3).

The "homology" in the above (V-2) is the same as those described for the above (I-3), (II-3), and (III-3).

At positions 34 to 36 of SEQ ID NO: 3 in (V-1) to (V-2) of the S-tag gene, a sequence encoding histidine present in a wild-type S-tag is substituted with a sequence encoding phenylalanine for the purpose of suppressing an influence on RNase activity caused by binding of the expressed S-tag to S protein in a host cell.

In addition, the binding position of the S-tag gene is not particularly limited, but from the viewpoint of reducing the influence on the activity of the enzyme to be fused with the S-tag, the S-tag can be designed to bind to the N-terminal of the enzyme (ZEP and/or CCS).

The linker gene is not particularly limited as long as the linker (peptide) has an appropriate distance between the S-tag and the enzyme in the fusion protein, thereby ensuring the degree of freedom between CCS and ZEP in the complex. A preferred sequence of the linker (peptide) in the fusion protein is a sequence in which a nonpolar amino acid and a polar amino acid are alternately linked from the viewpoint of eliminating polarity bias of the linker (peptide) itself and suppressing undesired adsorption and the like, and a combination of the nonpolar amino acid and the polar amino acid is preferably a combination of glycine and serine. The length of the linker (peptide) is, for example, 15 to 25 amino acids, preferably 18 to 22 amino acids.

As described above, the interposing position of the linker gene may be at least one of between the ZEP gene and the S-tag gene and between the CCS gene and the S-tag gene, but is preferably at least between the ZEP gene and the S-tag gene, and particularly preferably only between the ZEP gene and the S-tag gene (not interposed between the CCS gene and the S-tag gene).

### 1-4. Other genes

In addition to the sequences described above, the recombinant vector may contain regulatory sequences other than the first promoter and the second promoter. Such a regulatory sequence is positioned upstream (5' side), within, or downstream (3' side) of the sequence of the upstream gene of the carotenoid biosynthesis gene and/or the sequence of the downstream gene of the carotenoid biosynthesis gene, and affects transcription, RNA processing, stability, or the like. Examples of the regulatory sequence include a translation leader sequence, an intron, a polyadenylation recognition sequence, an RNA processing site, an effector binding site, a stem-loop structure, and the like.

### 1-5. Expression vector

As the expression vector, a vector constructed for gene recombination from a plasmid, a virus, a phage, a transposon, an IS element, a phasmid, a cosmid, or the like capable of autonomously growing in a host can be used. Examples of such expression vectors include pET, pLG, pACYC, pBR, pUC, pKC, pRep, pHS, pKK, pDHE, pPLc, pMBL, pUR, pIN, λgt, pBdCI, pUB, pC, and pBD.; pSA, pAJ, YEp, YRp, YIp, pYAC, pCDM, pMT2PC, an adenoviral vector, an adeno-associated viral vector, a retroviral vector, a herpesvirus vector, and the like. The underlying expression vectors may be the same or different between the first recombinant vector and the second recombinant vector. In addition, as the expression vector, an appropriate combination with a host cell may be selected and used, and for example, as a preferable example in the case of using E. coli as a host cell, a combination of a pACYC vector and a pUC vector with a JM101 (DE3) E. coli strain, and the like can be mentioned.

### 1-6. Host cells

The host is not particularly limited as long as the recombinant vector is stable, can autonomously proliferate, and can express the trait of a foreign gene, and may be a carotenoid-producing cell or a carotenoid-non-producing cell. Preferred examples of the host include bacteria belonging to the genus Escherichia such as Escherichia coli, etc., the genus Bacillus such as Bacillus subtilis, etc., the genus Pseudomonas such as Pseudomonas putida, etc., and the like; yeasts (for example, budding yeast (Saccharomyces cerevisiae), fission yeast (S. pombe), torula yeast (Candida utilis), methanol-assimilating yeast (Pichia pastoris), red yeast (Xanthophyllomyces dendrorhous), etc.), and the like, but may also include animal cells, insect cells, plants, and the like. Among them, E. coli is a particularly preferred host.

### 1-7. Production of transformant

The transformant of the present invention can be obtained by introducing a recombinant vector into a host, and conditions for introducing the recombinant vector into the host may be appropriately set according to the type of the host, and the like. When the host is a bacterium, examples thereof include a heat shock method, a method using a competent cell by calcium ion treatment, an electroporation method, and the like. When the host is a yeast, examples thereof include an electric boring method (electroporation method), a spheroplast method, a lithium acetate method, and the like. When the host is an animal cell, examples thereof include an electroporation method, a calcium phosphate method, a lipofection method, and the like. When the host is an insect cell, examples thereof include a calcium phosphate method, a lipofection method, an electroporation method, and the like. When the host is a plant cell, examples thereof include an electroporation method, an Agrobacterium method, a particle gun method, a PEG method, and the like.

Whether or not the recombinant vector has been incorporated into the host can be confirmed by a PCR method, a Southern hybridization method, a Northern hybridization method, or the like.

When it is confirmed whether or not the recombinant vector has been incorporated into the host by the PCR method, for example, the recombinant vector may be separated and purified from the transformant.

The separation and purification of the recombinant vector can be performed on the basis of a lysate obtained by lysing bacteria, for example, when the host is a bacterium. As a method of lysis, for example, treatment is performed with a lytic enzyme such as lysozyme or the like, and if necessary, a protease, other enzymes, and a surfactant such as sodium lauryl sulfate (SDS) or the like, are used in combination.

Furthermore, physical crushing methods such as freeze-thaw, ultrasonic irradiation and French press treatment may be combined. The separation and purification of DNA from the lysate can be performed, for example, by deproteinization by phenol treatment and protease treatment, ribonuclease treatment, alcohol precipitation treatment, and a commercially available kit being appropriately combined.

DNA cleavage can be performed according to a conventional method, for example, using restriction enzyme treatment. As the restriction enzyme, for example, a type II restriction enzyme that acts on a specific nucleotide sequence can be used. Binding between the DNA and the expression vector can be performed using, for example, a DNA ligase.

Thereafter, the separated and purified DNA is cut with a specific restriction enzyme, subjected to agarose gel electrophoresis, polyacrylamide gel electrophoresis, capillary electrophoresis, and the like, and stained with ethidium bromide, SYBR Green solution, and the like, and transformation can be confirmed from the detected band length pattern.

In addition, transformation can also be confirmed by designing primers and performing PCR using the isolated and purified DNA as a template. The amplification product obtained by PCR is subjected to agarose gel electrophoresis, polyacrylamide gel electrophoresis, capillary electrophoresis, or the like, stained with ethidium bromide, SYBR Green solution, or the like, and then the amplification product is detected as a band, whereby transformation can be confirmed.

In addition, PCR can be performed using a primer labeled with a fluorescent dye or the like in advance to detect an amplification product. Furthermore, a method of binding an amplification product to a solid phase such as a microplate or the like and confirming the amplification product by fluorescence, an enzymatic reaction, or the like may also be adopted.

### 2. Method for producing carotenoid composition

The method for producing the carotenoid composition of the present invention can be obtained by a production method including a step of culturing the transformant of the present invention.

The culture conditions of the transformant may be appropriately set in consideration of the nutritional physiological properties of the host, and liquid culture is preferable. In the case of industrial production, aeration stirring culture is preferable.

As a nutrient source of the medium, those required for growth of transformants can be used. The carbon source may be any carbon compound that can be assimilated, and examples thereof include molasses, glucose, fructose, maltose, sucrose, lactose, sucrose, starch, lactose, glycerol, pyruvic acid, and the like.

The nitrogen source may be any assimilable nitrogen compound, and examples thereof include natural components such as corn steep liquor, peptones (meat peptone, casein peptone, soybean peptone, etc.), extracts (meat extract, yeast extract), and the like; ammonium salts such as ammonium acetate, ammonium chloride, ammonium sulfate, etc., amino acids such as glutamic acid, aspartic acid, glycine, etc.; and the like.

In addition to the carbon source and the nitrogen source, for example, phosphates, carbonates, sulfates, salts of magnesium, calcium, potassium, iron, manganese, zinc and the like, specific vitamins, and the like may be used as necessary.

The culture temperature can be appropriately set within a range in which the transformant of the present invention can grow and the transformant of the present invention produces the polypeptide of the present invention, but it is preferably about 20 to 40°C, preferably about 30 to 37°C. The culturing may be completed at an appropriate time with reference to the time when the polypeptide of the present invention reaches the highest yield, and examples of the culturing time include 24 to 200 hours, preferably about 60 to 90 hours.

Examples of the method for recovering the bacterial cells after completion of the culture include filtration, decantation, and centrifugation of the culture solution, and the like. The recovered bacterial cells can be treated with water, a sodium chloride solution, dimethylformamide, or the like as necessary.

The carotenoid composition obtained in the production method of the present invention typically contains capsanthin, preferably further contains capsorubin, capsanthin 3'-acetate, capsorubin 3-acetate, cucurbitaxanthin A, capsorubin diacetate, capsanthin 3,6-epoxide, and/or capsanthin 3,6-epoxide 3'-acetate.

For the extraction of the carotenoid composition from the bacterial cells, an appropriate organic solvent can be used. Examples of the organic solvent include methanol, ethanol, isopropyl alcohol, acetone, hexane, diethyl ether, tetrahydrofuran, methyl ethyl ketone, methyl isobutyl ketone, dichloromethane, chloroform, dimethylformamide, dimethyl sulfoxide, methyl acetate, ethyl acetate, and the like, and one or more of these solvents can be selected. Among these organic solvents, acetone, methanol, and diethyl ether are preferable.

The carotenoid composition fraction obtained as described above may be directly concentrated and dried as a composition, or each carotenoid can be purified, concentrated, and dried.

The purification method can be performed, for example, by appropriately combining methods such as gel filtration, adsorption chromatography, ion exchange chromatography, affinity chromatography, and the like. Examples of the drying method include freeze drying, vacuum drying, spray drying, and the like.

### EXAMPLES

Hereinafter, the present invention will be described more specifically with reference to examples, but the present invention is not limited thereto. The promoter used in the following Examples and Comparative Examples includes Plac, promoters unique to the carotenoid biosynthesis gene (promoters naturally present in Pantoea ananatis, specifically promoters included in DDBJ accession number D90087), PBAD, or Ptac, and the relationship of promoter intensity is Plac < promoter unique to the carotenoid biosynthesis gene < PBAD < Ptac, and the promoter intensity of PBAD is three times or more the promoter intensity of the promoter unique to the carotenoid biosynthesis gene.

### [Comparative Example 1]

In this comparative example, pACHP-Zea shown in Fig. 2 was constructed as the first recombinant vector to be incorporated, and pUC-Plac-CaCCS-CaZEP shown in Fig. 2 was constructed as the second recombinant vector. These recombinant vectors were designed so that the expression level of upstream gene was relatively higher by the promoter unique to the carotenoid biosynthesis gene (derived from Pantoea ananatis), and the expression level of downstream gene was relatively lower by Plac. An outline of the first recombinant vector and the second recombinant vector of the present comparative example is shown in Table 1.

**[Table 1]**

| | Recombinant vector | Promoter | Promoter relative intensity | Vector constitution (abbreviation) |
|---|---|---|---|---|
| Comparative Example 1 | First | Derived from pantoea ananatis | High | pACHP-Zea |
| | Second | Plac | Low | pUC-Plac-CaCCS-CaZEP |

pACHP-Zea as the first recombinant vector is a vector for zeaxanthin synthesis used in the report of Non-Patent Document 3. pACHP-Zea is configured by inserting, into a pACYC vector, an idi gene derived from Haematococcus pluvalis (DDBJ Accession No. AB019034) and a gene group required for zeaxanthin biosynthesis (crtE, crtY, crtI, crtB, and crtZ genes derived from Pantoea ananatis (DDBJ accession number D90087)).

pUC-Plac-CaCCS-CaZEP as the second recombinant vector was produced by placing Capsicum annuum-derived ZEP gene (hereinafter, it is described as "CaZEP"; DDBJ accession number XM_016705616) and Capsicum annuum-derived CCS gene (hereinafter, referred to as "CaCCS"; DDBJ Accession No. X76165) downstream of the Plac-promoter of the pUC vector.

Both the first recombinant vector and the second recombinant vector were introduced into the E. coli JM109 strain by a heat shock method.

After introduction of the recombinant vector, single colonies on the LB medium were transplanted into 5 mL of LB liquid medium, and cultured with shaking at 200 rpm at 37°C for 16 hours. A volume of 100 µL of the culture medium was transplanted into 10 mL of TB liquid medium, and after 24 hours of shaking culture at 200 rpm at 30°C, 10 µL of isopropyl-β-D-galactopyranoside (IPTG) was added to induce gene expression, and the culture was continued for 48 hours. Chloramphenicol (30 mg/L) and kanamycin (40 mg/L) as antibiotics were added to the medium.

A volume of 1 mL of the obtained culture solution was centrifuged at 8,000 g for 1 minute, and the supernatant was removed to collect a cell fraction. The cell fraction was suspended in 1% sodium chloride solution and centrifuged at 8,000 g for 1 minute, the supernatant was removed, and the cells were collected. To the collected cells was added 0.5 mL of acetone, 1 mL of diethyl ether/hexane (1 : 1 by volume) and vortexed vigorously. After 1 mL of water was added and stirred, the mixture was allowed to stand for 5 minutes, and the upper layer was collected in a 1.5 mL centrifugal tube and dried and solidified by a vacuum centrifugal concentrator.

The resulting dried solid was subjected to separation of carotenoids using Aquity H-class UPLC (Waters). Using Aquity UPLC BEH C18 column (2.1 × 100 mm, 1.7 µm, Waters) as a column, acetonitrile : ultrapure water (85 : 15 (volume basis)) as a mobile phase A, and acetonitrile : methanol (65 : 35 (volume basis)) as a mobile phase B, the mobile phase A and the mobile phase B were fed by gradient for 8 minutes such that the ratio of the mobile phase A : the mobile phase B was changed from 100 : 0 to 0 : 100. Thereafter, the carotenoid was separated by holding the ratio of 0 : 100 for 5 minutes.

As a result, a peak having the same elution time as that of the capsanthin sample could not be confirmed.

### [Comparative Example 2 (Comparative Example 2-1 and Comparative Example 2-2)]

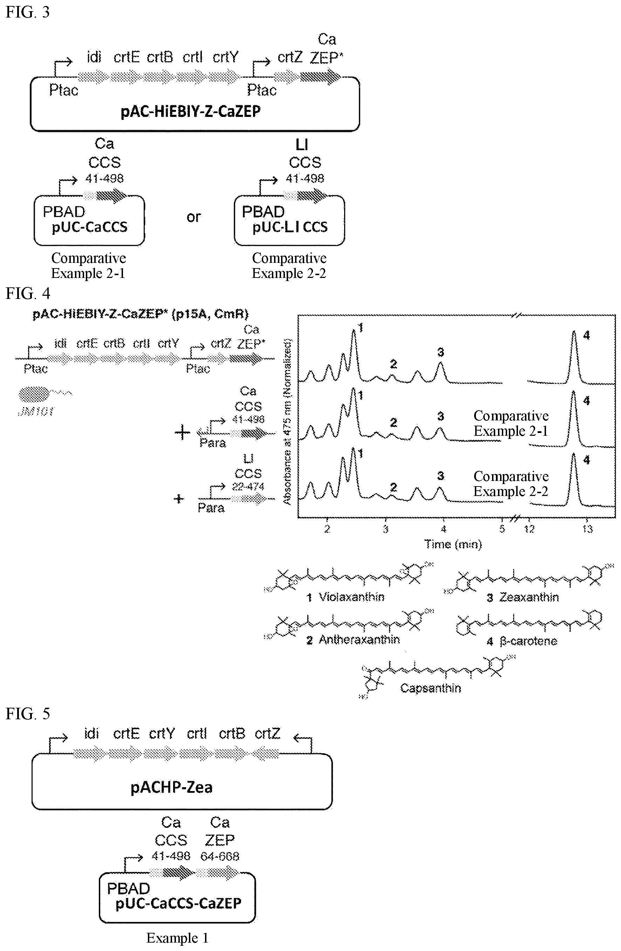

In this comparative example, pAC-HiEBIY-Z-CaZEP shown in Fig. 3 was constructed as the first recombinant vector to be incorporated, and pUC-CaCCS (The case of using this second recombinant vector is defined as Comparative Example 2-1.) or pUC-LlCCS (The case of using this second recombinant vector is defined as Comparative Example 2-2.) shown in Fig. 3 was constructed as the second recombinant vector. These recombinant vectors were designed so that the expression level of ZEP gene among upstream genes and downstream genes was relatively higher by Ptac, and the expression level of CCS gene among downstream genes was relatively lower by PBAD. An outline of the first recombinant vector and the second recombinant vector of the present comparative example is shown in Table 2.

**[Table 2]**

| | Recombinant vector | Promoter | Promoter relative intensity | Vector constitution (abbreviation) |
|---|---|---|---|---|
| Comparative Example 2-1 | First | Ptac | High | pAC-HiEBIY-Z-CaZEP^{(∗1)} (^{∗}1: CaZEP₆₄₋₆₆₈) |
| | Second | PBAD | Low | pUC-CaCCS^{(∗2)} (^{∗}2: CaCCS₄₁₋₄₉₈) |
| Comparative Example 2-2 | First | Ptac | High | pAC-HiEBIY-Z-CaZEP^{(∗1)} (^{∗}1: CaZEP₆₄₋₆₆₈) |
| | Second | PBAD | Low | pUC-LICCS^{(∗2)} (^{∗}2: LICCS₄₁₋₄₉₈) |

In Comparative Example 2-1 and Comparative Example 2-2, pAC-HiEBIY-Z-CaZEP in which the Haematococcus pluvalis-derived idi gene, and together, the respective genes crtE, crtB, crtI, crtY, and crtZ derived from Pantoea ananatis necessary for biolaxanthin biosynthesis, and the 64th to 668th amino acid residues (This is because a plant-derived gene often has a signal sequence unnecessary in E. coli at the N-terminal of a protein. It is abbreviated as CaZEP₆₄₋₆₆₈ and is denoted as ZEP* in Fig. 3.) of CaZEP were placed downstream of the Ptac promoter, in a pACYC plasmid vector, was produced as a first recombinant vector on the basis of the report of Non-Patent Document 3.

The produced first recombinant vector pAC-HiEBIY-Z-CaZEP was introduced into E. coli JM101 (DE3) and cultured in the same manner as in Comparative Example 1 to obtain a supernatant.

The obtained supernatant was subjected to separation of carotenoids using HPLC Chromaster system manufactured by Hitachi, Ltd. Using Inertsil ODS-3 (4.6 × 150 mm, 5 µm, GL Sciences) as a column, acetonitrile as a mobile phase A, and isopropanol as a mobile phase B, the mobile phase A and the mobile phase B were fed at the ratio of 100 : 0 for 5 minutes, and then the mobile phase A and the mobile phase B were fed by gradient for 5 minutes such that the ratio of the mobile phase A : the mobile phase B was changed from 100 : 0 to 50 : 50 (volume basis), and the ratio of 50 : 50 was further held for 5 minutes to separate carotenoids.

Fig. 4 shows the obtained HPLC chromatogram. As shown in Fig. 4, it was confirmed that zeaxanthin and β-carotene and many other carotenoid intermediates were produced in addition to violaxanthin and antheraxanthin.

Next, in Comparative Example 2-1 among Comparative Example 2-1 and Comparative Example 2-2, it was examined whether capsanthin and capsorubin could be produced by co-expressing CaCCS in this carotenoid producing E. coli strain. The N-terminal chloroplast signal sequence of CaCCS was predicted, and CaCCS₄₁₋₄₉₈ with the N-terminal amino acid deleted was designed. Plasmid pUC-CaCCS, in which CaCCS₄₁₋₄₉₈ was placed downstream of the PBAD promoter of the pUC vector, was prepared as a second recombinant vector and introduced into the carotenoid producing E. coli strain.

Further, in Comparative Example 2-2 among Comparative Example 2-1 and Comparative Example 2-2, pUC-LlCCS was produced as a second recombinant vector in the same manner as in Comparative Example 2-1, except that Lilium lancifoliumderived CCS (hereinafter referred to as "LlCCS"; DDBJ Accession No. GU443955) was introduced instead of CaCCS, and introduced into the above carotenoid-producing E. coli strain.

In Comparative Example 2-1 and Comparative Example 2-2, after introduction of the respective second recombinant vectors, the single colonies on the LB medium were transplanted into 3 mL of LB liquid medium, and 100 µL of the culture solution subjected to shaking culture at 200 rpm and 37°C for 16 hours was transplanted into 10 mL of TB liquid medium, and after shaking culture at 200 rpm and 30°C for 24 hours, 10 µL of isopropyl-β-D-galactopyranoside (IPTG) was added to induce gene expression, and further the culture was continued for 48 hours. Chloramphenicol (30 mg/L) and kanamycin (40 mg/L) as antibiotics were added to the medium.

A volume of 1 mL of the obtained culture solution was centrifuged at 8,000 g for 1 minute, and the supernatant was removed to collect a cell fraction. The cell fraction was suspended in 1% sodium chloride solution and centrifuged at 8,000 g for 1 minute, the supernatant was removed, and the cells were collected. Acetone/methanol (7 : 3 (volume basis)) were added to the recovered cells to extract the carotenoid, centrifuged at 12,000 g for 2 minutes, and the supernatant was used for carotenoid analysis.

The obtained supernatant was subjected to separation of carotenoids using HPLC Chromaster system manufactured by Hitachi, Ltd. Using Inertsil ODS-3 (4.6 × 150 mm, 5 µm, GL Sciences) as a column, acetonitrile as a mobile phase A, and isopropanol as a mobile phase B, the mobile phase A and the mobile phase B were fed at the ratio of 100 : 0 for 5 minutes, and then the mobile phase A and the mobile phase B were fed by gradient for 5 minutes such that the ratio of the mobile phase A : the mobile phase B was changed from 100 : 0 to 50 : 50 (volume basis), and the ratio of 50 : 50 was further held for 5 minutes.

As a result, as shown in the results of Comparative Example 2-1 and Comparative Example 2-2 in Fig. 4, when either CaCCS or LlCCS were co-expressed, there was no change in the produced carotenoid, and production of capsanthin could not be confirmed.

### [Example 1]

In this example, pACHP-Zea shown in Fig. 5 was constructed as the first recombinant vector to be incorporated, and pUC-CaCCS-CaZEP shown in Fig. 5 was constructed as the second recombinant vector. These recombinant vectors were designed such that the expression level of upstream gene was relatively low due to the promoter unique to the carotenoid biosynthesis gene (derived from Pantoea ananatis), and the expression level of downstream gene was relatively high due to PBAD. An outline of the first recombinant vector and the second recombinant vector of this example is shown in Table 3.

**[Table 3]**

| | Recombinant vector | Promoter | Promoter relative intensity | Vector constitution (abbreviation) |
|---|---|---|---|---|
| Example 1 | First | Derived from pantoea ananatis | Low | pACHP-Zea |
| | Second | PBAD | High | pUC-CaCCS^{(∗2)}-CaZEP^{(∗1)} (^{∗}2: CaCCS₄₁₋₄₉₈)(^{∗}1: CaZEP₆₄₋₆₆₈) |

pACHP-Zea as the first recombinant vector was prepared in the same manner as in Comparative Example 1. According to the report of Non-Patent Document 3, regarding this pACHP-Zea, it has been found that β-carotene is not detected in the carotenoid composition with the transformant, and zeaxanthin occupies the total carotenoid.

pUC-CaCCS-CaZEP as the second recombinant vector was generated by placing CaZEP₆₄₋₆₆₈ and CaCCS₄₁₋₄₉₈ downstream of the PBAD promoter of the pUC vector.

Both the first recombinant vector and the second recombinant vector were introduced into E. coli JM101 (DE3) in the same manner as in Comparative Example 1.

After introduction of the recombinant vector, a single colony on the LB medium was transplanted into 3 mL of LB liquid medium, and cultured with shaking at 200 rpm at 37°C for 16 hours. A volume of 100 µL of the culture medium was transplanted into 10 mL of TB liquid medium, and after 24 hours of shaking culture at 200 rpm at 30°C, 10 µL of isopropyl-β-D-galactopyranoside (IPTG) was added to induce gene expression, and the culture was continued for 48 hours. Chloramphenicol (30 mg/L) and kanamycin (40 mg/L) as antibiotics were added to the medium.

A volume of 1 mL of the obtained culture solution was centrifuged at 8,000 g for 1 minute, and the supernatant was removed to collect a cell fraction. The cell fraction was suspended in 1% sodium chloride solution and centrifuged at 8,000 g for 1 minute, the supernatant was removed, and the cells were collected. Acetone/methanol (7 : 3 by volume) were added to extract the carotenoid, centrifuged at 12,000 g for 2 minutes and the supernatant was used for carotenoid analysis.

The obtained supernatant was subjected to separation of carotenoids using HPLC Chromaster system manufactured by Hitachi, Ltd. Using Inertsil ODS-3 (4.6 × 150 mm, 5 µm, GL Sciences) as a column, acetonitrile as a mobile phase A, and isopropanol as a mobile phase B, the mobile phase A and the mobile phase B were fed at the ratio of 100 : 0 for 5 minutes, and then the mobile phase A and the mobile phase B were fed by gradient for 5 minutes such that the ratio of the mobile phase A : the mobile phase B was changed from100 : 0 to 50 : 50 (volume basis), and the ratio of 50 : 50 was further held for 5 minutes to separate carotenoids.

The obtained supernatant (carotenoid) was also subjected to Aquity H-class UPLC (Waters). Using Aquity UPLC BEH C18 column (2.1 × 100 mm, 1.7 µm, Waters) as a column, acetonitrile : ultrapure water (85 : 15 (volume ratio)) as a mobile phase A, and acetonitrile : methanol (65 : 35 (volume ratio)) as a mobile phase B, the mobile phase A and mobile phase C were fed by gradient for 8 minutes such that the ratio of A : B was changed from 100 : 0 to 0 : 100, and then the carotenoid was separated by a program of holding at the ratio of 0 : 100 for 5 minutes.

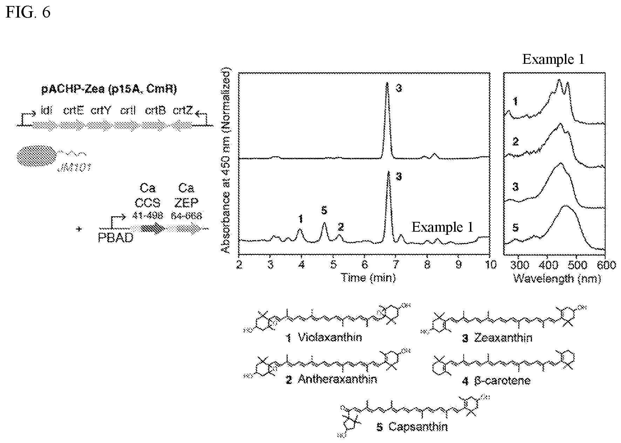

The results are shown in Fig. 6. Fig. 6 shows the results of the HPLC chromatogram when the first expression vector is expressed alone (upper part) and the HPLC chromatogram when the first expression vector and the second expression vector are co-expressed (Example 1) (lower part), and the absorption spectra of peaks 1, 2, 3, and 5 detected in the latter (Example 1). As shown in Fig. 6, a new peak 5 could be confirmed by the co-expression of the first expression vector and the second expression vector.

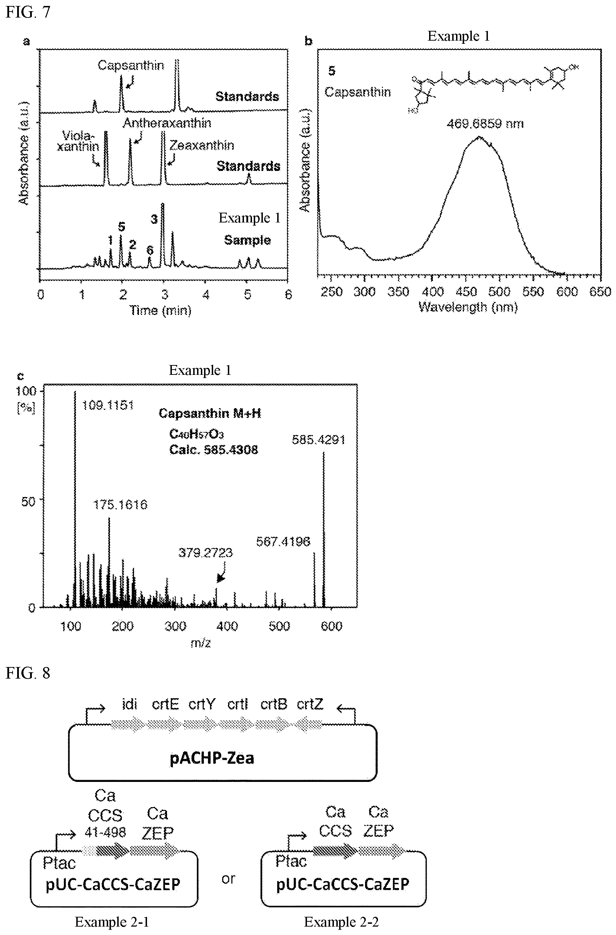

The same supernatant (carotenoid)(sample) was analyzed with UPLC and the results compared to standards are shown in Figure 7a. As shown in Fig. 7a, a new peak 5 could be confirmed by co-expression of the first expression vector and the second expression vector (Example 1). The absorption spectrum of the peak 5 is shown in Fig. 7b. The peak 5 confirmed in the UPLC chromatogram of Fig 7a showed a retention time and absorption spectrum similar to those of capsanthin. As a result of calculation based on the molar absorption coefficient from the peak intensity, the production amount of capsanthin per culture solution was 292 µg/L.

LC/MS analysis of the peak 5 was performed using a Waters Xevo G2S Q TOF mass spectrometer (Waters) equipped with the Acquity UPLC system. As analysis conditions with a time-of-flight mass spectrometer (ESI-TOF-MS), a nitrogen gas was used at a capillary voltage of 3.2 kV, a cone voltage of 20 eV, 120°C, and 30 L/h, and m/z of 100 to 1,500 was scanned. MS/MS spectra were measured on a quadrupole TOF MS/MS instrument using argon at a collision energy of 20 V. UV-VIS absorption spectra were recorded between 200 nm and 600 nm using a photodiode array. HPLC separation used Acquity 1.7 µm BEH UPLC C18 column (Waters) with a gradient program of acetonitrile: ultrapure water (85 : 15 by volume) to acetonitrile : methanol (65 : 35 by volume) (flow rate 0.4 mL/min, 15 minutes). The results are shown in Fig. 7c. The molecular ion peak shown in Fig. 7c coincided with the calculated value of the molecular weight of capsanthin.

The ¹H NMR (500 MHz) spectrum of the peak 5 was measured using a Varian UNITY INOVA 500 spectrometer (Varian Corporation) using TMS as an internal standard and using deuterated chloroform. As a result, the peak 5 was identified as capsanthin.

### [Example 2 (Example 2-1 and Example 2-2)]

In this example, pACHP-Zea shown in Fig. 8 was constructed as the first recombinant vector to be incorporated, and pUC-CaCCS-CaZEP (two kinds) shown in Fig. 8 was constructed as the second recombinant vector. These recombinant vectors were designed so that the expression level of the upstream gene was relatively low by the promoter unique to the carotenoid biosynthesis gene (derived from Pantoea ananatis), and the expression level of the downstream gene was relatively high by Ptac. An outline of the first recombinant vector and the second recombinant vector of this example is shown in Table 4.

**[Table 4]**

| | Recombinant vector | Promoter | Promoter relative intensity | Vector constitution (abbreviation) |
|---|---|---|---|---|
| Example 2-1 | First | Derived from Pantoea ananatis | Low | pACHP-Zea |
| | Second | Ptac | High | pUC-CaCCS^{(∗2)}-CaZEP (^{∗}2: CaCCS₄₁₋₄₉₈) |
| Example 2-2 | First | Derived from Pantoea ananatis | Low | pACHP-Zea |
| | Second | Ptac | High | pUC-CaCCS-CaZEP |

pACHP-Zea as the first recombinant vector was prepared in the same manner as in Comparative Example 1. According to the report of Non-Patent Document 3, regarding this pACHP-Zea, it has been found that β-carotene is not detected in the carotenoid composition with the transformant, and zeaxanthin occupies the total carotenoid.

The pUC-CaCCS-CaZEP as the second recombinant vector was produced by placing CaZEP (signal sequence not removed) and CaCCS (signal sequence not removed) or CaCCS₄₁₋₄₉₈ downstream of the Ptac promoter of the pUC vector.

Both the first recombinant vector and the second recombinant vector were introduced into E. coli JM101 (DE3) in the same manner as in Comparative Example 1.

After introduction of the recombinant vector, a single colony on the LB medium was transplanted into 0.5 mL of LB liquid medium, and cultured with shaking at 1,000 rpm at 37°C for 16 hours. A volume of 20 µL of the culture medium was transplanted into 2 mL of TB liquid medium, and after shaking culture at 1,000 rpm at 30°C for 24 hours, 2 µL of 1 M isopropyl-β-D-galactopyranoside (IPTG) was added to induce gene expression. The cells were further cultured for 48 hours. As antibiotics, chloramphenicol (30 mg/L) and kanamycin (40 mg/L) were added to the medium.

A volume of 1 mL of the obtained culture solution was centrifuged at 8,000 g for 1 minute, and the supernatant was removed to collect a cell fraction. The cell fraction was suspended in 1% sodium chloride solution and centrifuged at 8,000 g for 1 minute, the supernatant was removed, and the cells were collected. Acetone/methanol (7 : 3 by volume) were added to extract the carotenoid, centrifuged at 12,000 g for 2 minutes and the supernatant was used for carotenoid analysis.

The obtained supernatant was subjected to separation of carotenoids using HPLC Chromaster system manufactured by Hitachi, Ltd. Using Inertsil ODS-3 (4.6 × 150 mm, 5 µm, GL Sciences) as a column, acetonitrile as a mobile phase A, and isopropanol as a mobile phase B, the mobile phase A and the mobile phase B were fed at the ratio of 100 : 0 for 5 minutes, and then the mobile phase A and the mobile phase B were fed by gradient for 5 minutes such that the ratio of the mobile phase A : the mobile phase B was changed from100 : 0 to 50 : 50 (volume basis), and the ratio of 50 : 50 was further held for 5 minutes to separate carotenoids.

For comparison, the same operation was performed using a second expression vector for comparison produced in the same manner except that neither CaCCS nor CaCCS₄₁₋₄₉₈ was incorporated into the second expression vector described above.

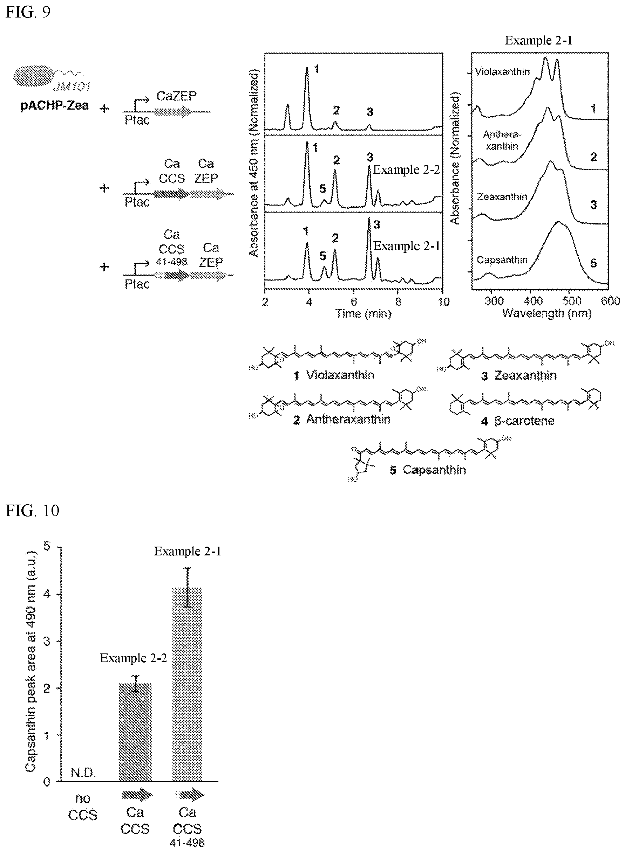

The analysis results are shown in Fig. 9. Fig. 9 shows the results of an HPLC chromatogram in the case of co-expressing the first expression vector and the second expression vector for comparison (upper part), an HPLC chromatogram in the case of co-expressing the first expression vector and the second expression vector (one incorporating full-length CaCCS: Example 2-2) (middle part), and an HPLC chromatogram in the case of co-expressing the first expression vector and the second expression vector (one incorporating CaCCS₄₁₋₄₉₈ with a signal sequence removed: Example 2-1) (lower part), and absorption spectra of peaks 1, 2, 3, and 5 detected in the case of co-expressing the first expression vector and the second expression vector (one incorporating CaCCS₄₁₋₄₉₈ with a signal sequence removed: Example 2-1). As shown in Fig. 9, a new peak 5, that is, capsanthin could be confirmed by co-expression of the first expression vector and the second expression vector.

In addition, Fig. 10 shows a graph comparing the intensities of the peaks 5 of capsanthin in Fig. 9. As shown in Fig. 10, capsanthin was produced more in the case of using the second expression vector incorporating CCS₄₁₋₄₉₈ from which the signal sequence was removed (Example 2-1) than in the case of using the second expression vector incorporating the full-length CCS (Example 2-2).

Furthermore, as a result of calculating the production amount of capsanthin of the peak 5 from the peak intensity based on the molar absorption coefficient, the production amount of capsanthin per culture medium in the case of using the second expression vector incorporating the full-length CCS (Example 2-2) was 326 µg/L, and the production amount of capsanthin per culture medium in the case of using the second expression vector incorporating CCS₄₁₋₄₉₈ from which the signal sequence has been removed (Example 2-1) was 452 µg/L.

### [Example 3 (Example 3-1, Example 3-2, Example 3-3)]

In this example, the same pACHP-Zea as in Comparative Example 1 was used as the first recombinant vector. According to the report of Non-Patent Document 3, regarding this pACHP-Zea, it has been found that β-carotene is not detected in the carotenoid composition with the transformant, and zeaxanthin occupies the total carotenoid.

As the second recombinant vector, pUC-S/slC/sZ, pUC-S/sC/slZ, or pUC-S/slC/slZ ("S" represents S Protein, "s" represents S-Tag, and "l" represents a linker.) shown in Fig. 11 was produced. An outline of the first recombinant vector and the second recombinant vector of this example is shown in Table 5.

**[Table 5]**

| | Recombinant vector | Promoter | Promoter relative intensity | Vector constitution (For the second recombinant vector, the upper row shows the abbreviations and the lower row shows the detailed structure) |
|---|---|---|---|---|
| Example 3-1 | First | Derived from Pantoea ananatis | Low | pACHP-Zea |
| | Second | Ptac | High | pUC-S/slC/sZ |
| | | | | S protein / s tag + linker + CaCCS₄₁₋₄₉₈ / s tag + CaZEP |
| Example 3-2 | First | Derived from Pantoea ananatis | Low | pACHP-Zea |
| | Second | Ptac | High | S/sC/slZ |
| | | | | S protein / s tag + CaCCS₄₁₋₄₉₈ / s tag + linker + CaZEP |
| Example 3-3 | First | Derived from Pantoea ananatis | Low | pACHP-Zea |
| | Second | Ptac | High | S/slC/slZ |
| | | | | S protein / s tag + linker + CaCCS₄₁₋₄₉₈ / s tag + linker + CaZEP |

As schematically shown in Fig. 12, this second recombinant vector was designed so that CCS and ZEP were expressed as proteins fused with S-Tag (In Fig. 12, illustrated as "S tag + linker + CCS" and "S tag + linker + ZEP"), respectively, and then indirectly bound via multimerized S Protein (In Fig. 12, it is indicated as "multimer".) to form a complex (In Fig. 12, it is indicated as "complex".). An aspect of the second recombinant vector designed as described above is also referred to as "S-Tag incorporated".

The three second recombinant vectors of pUC-S/slC/sZ, pUC-S/sC/slZ, and pUC-S/slC/slZ shown in Fig. 11 were designed so that the combination of the presence or absence of a linker between S-Tag and CCS and/or between S-Tag and ZEP was different. By making the introduction position of the linker in the second recombinant vector into which S-Tag was incorporated different in this way, three conditions in which the degree of freedom between CCS and ZEP was different after expression were constructed.

Sequence information on construction of the second recombinant vector into which S-Tag is incorporated is shown in Table 6 below.

**[Table 6]**

| | Base sequence | Amino acid sequence |
|---|---|---|
| S protein | SEQ ID NO: 1 | SEQ ID NO: 2 |
| S-Tag | SEQ ID NO: 3 | SEQ ID NO: 4 |
| S-Tag/CaCCS | SEQ ID NO: 5 | SEQ ID NO: 6 |
| S-Tag/linker/CaCCS | SEQ ID NO: 7 | SEQ ID NO: 8 |
| S-Tag/CaZEP | SEQ ID NO: 9 | SEQ ID NO: 10 |
| S-Tag/linker/CaZEP | SEQ ID NO: 11 | SEQ ID NO: 12 |

As shown in SEQ ID NOs: 8 and 12, a linker consisting of 20 amino acid residues ({GS}₁₀ linker) in which 10 units of a unit (GS) consisting of glycine (G) of a nonpolar amino acid and serine (S) of a polar amino acid were continuous was used as the linker. The fusion position of S-Tag was the N-terminus of each of CaCCS and CaZEP. As a construct into which S protein, S-Tag, or the like is introduced, pUClac-ptac-CaCCSM40-CaZEP (pMF541) (Furubayashi, M., Kubo, A., Takemura, M., Otani, Y., Maoka, T., Terada, Y., Yaoi, K., Ohdan, K., Misawa, N. and Mitani, Y. (2021) J.Agric.Food Chem., 69, 5076-5085) was used.

The first recombinant vector and the second recombinant vector were introduced in the same manner as in Example 1, then a single colony on the LB medium was transplanted into 3 mL of a 2YT liquid medium, and cultured with shaking at 105 rpm and 28°C for 16 hours using Shaking Bath BW201 manufactured by Yamato Scientific Co., Ltd. A volume of 20 µL of the culture solution was transplanted into 2.1 mL of TB liquid medium, and after shaking culture was performed at 180 rpm and 30°C for 4 hours using Lab-Therm LT-X manufactured by Kuhner, 0.1 mM isopropyl-β-D-galactopyranoside (IPTG) was added to induce gene expression, and the culture was continued for 48 hours. As antibiotics, chloramphenicol (30 mg/L) and ampicillin (100 mg/L) were added to the medium.

A volume of 1 mL of the obtained culture solution was centrifuged at 8,000 g for 1 minute, and the supernatant was removed to collect a cell fraction. The cell fraction was suspended in 1% sodium chloride solution and centrifuged at 8,000 g for 1 minute, the supernatant was removed, and the cells were collected. Methanol and chloroform were added to extract the carotenoid, centrifuged at 12,000 g for 2 minutes, and the supernatant was used for carotenoid analysis.

The obtained supernatant was subjected to separation of carotenoids using HPLC Chromaster system manufactured by Hitachi, Ltd. Using Inertsil ODS-3 (4.6 × 150 mm, 5 µm, GL Sciences) as a column, acetonitrile as a mobile phase A, and isopropanol as a mobile phase B, the mobile phase A and the mobile phase B were fed at the ratio of 100 : 0 for 5 minutes, and then the mobile phase A and the mobile phase B were fed by gradient for 5 minutes such that the ratio of the mobile phase A : the mobile phase B was changed from100 : 0 to 50 : 50 (volume basis), and the ratio of 50 : 50 was further held for 5 minutes to separate carotenoids.

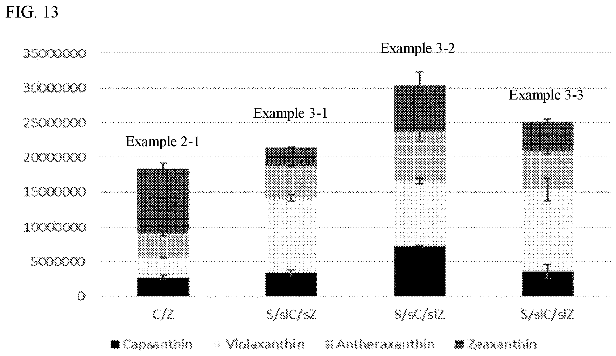

A graph comparing the peak intensities of the carotenoids in the HPLC analysis is shown in Fig. 13. In x Fig. 13, results in the case of using the second recombinant vector pUC-CaCCS₄₁₋₄₉₈-CaZEP (in the figure, it is indicated as "C/Z".) used in Example 2-1 in which S-Tag is not incorporated are also shown together. As shown in Fig. 13, more capsanthin was accumulated in the case of using the second recombinant vector into which S-Tag was incorporated (Example 3-1 to Example 3-3) than in the case where S-Tag was not incorporated (Example 2-1). In addition, among the three types of second recombinant vectors in which S-Tag was incorporated, when pUC-S/sC/slZ in which a linker was incorporated between S-Tag and ZEP was used (Example 3-2), the accumulation amount of capsanthin was remarkably large.

Further, the results calculated from the peak intensity based on the molar absorption coefficient are shown in Table 7. The production amount of capsanthin per culture medium in the case of using the second recombinant vector into which the S-Tag was incorporated (Examples 3-1 to 3-3) was improved as compared with the production amount of capsanthin per culture medium in the case of using the second recombinant vector into which the S-tag was not incorporated (Example 2-1). In particular, when pUC-S/sC/slZ in which a linker was incorporated between S-Tag and ZEP was used as the second recombinant vector (Example 3-2), not only the production amount of capsanthin per culture solution was remarkably improved, but also the ratio of capsanthin to the total amount of carotenoids shown in Table 7 was remarkably improved.

**[Table 7]**

| | Example 2-2 | | Example 3-1 | | Example 3-2 | | Example 3-3 | |
|---|---|---|---|---|---|---|---|---|
| | C/Z | | S/sIC/sZ | | S/sC/sIZ | | S/sIC/sIZ | |
| | ug/gFW | ug/L | ug/gFW | ug/L | ug/gFW | ug/L | ug/gFW | ug/L |
| Zeaxanthin | 3.88 | 1151.7 | 1.58 | 334.44 | 3.57 | 817.68 | 2.53 | 522.86 |
| Antheraxanthin | 1.48 | 440.41 | 2.72 | 574.75 | 3.91 | 894.03 | 3.35 | 692.94 |
| Violaxanthin | 0.98 | 289.22 | 5.28 | 1113.7 | 4.27 | 977.97 | 5.93 | 1225.5 |
| Capsanthin | 1.16 | 343.41 | 2.04 | 431.13 | 3.94 | 901.4 | 2.19 | 452.13 |
| Total | 7.5 | 2224.7 | 11.62 | 2454.1 | 15.7 | 3591.1 | 14 | 2893.4 |
| Capsanthin/Total (%) | 15.5 | | 17.6 | | 25.1 | | 15.6 | |

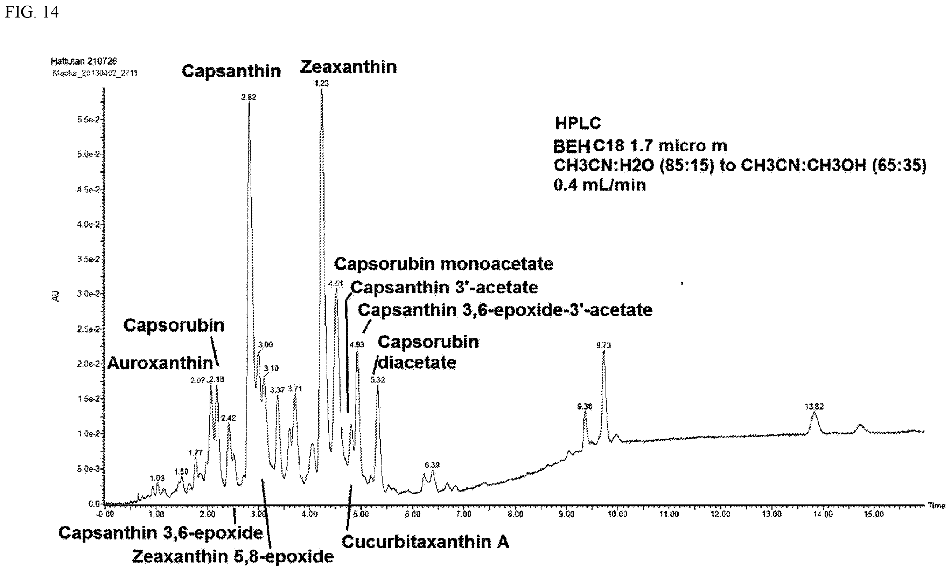

In addition, Fig. 14 shows the results of UPLC analysis in the same manner as in Example 1 of the carotenoids obtained when pUC-S/sC/slZ was used as the second vector (Example 3-2). As is clear from Fig. 14, the respective peaks of capsanthin 3'-acetate, capsanthin 3,6-epoxide, capsanthin 3,6-epoxide 3'-acetate, capsorubin 3-acetate, capsorubin diacetate, and cucurbitaxanthin A were detected. As a result of LC/MS analysis of these carotenoids in the same manner as in Example 1, the molecular ion peak coincided with the calculated value of the molecular weight of each carotenoid. Furthermore, the results of calculating the ratio of each carotenoid from the peak intensity of the UPLC analysis are shown in Table 8.

**[Table 8]**

| Carotenoids | % |
|---|---|
| β-Carotene | 3.3 |
| β-Cryptoxanthin | 3.7 |
| Capsorubin diacetate | 4.1 |
| Capsanthin 3,6-epoxide-3'-acetate | 5.5 |
| Cucurbitaxanthin A | 2.8 |
| Capsanthin 3'-acetate | 3.5 |
| Capsorubin monoacetate | 12.1 |
| Zeaxanthin | 19.4 |
| Zeaxanthin epoxide | 9.4 |
| Capsanthin | 18.5 |
| Capsanthin 3,6-epoxide | 3.2 |
| Capsorubin | 3.7 |
| Auroxanthin | 3.8 |
| Others | 7.0 |
| | 100.0 |

For NMR measurement of the carotenoid, silica gel chromatography was used, and the polarity was sequentially increased from hexane to ether : hexane (1 : 1), and ether : acetone (1 : 1), each fraction was fractionated and concentrated, then further, a silica gel (Cosmosil 5 SL-II) column 10 × 250 mm was used, acetone : hexane (3 : 7) was used as a mobile phase, and separated at a flow rate of 2 ml/min to obtain a sample from which each carotenoid peak had been fractionated, and the sample was used for ¹H NMR measurement in the same manner as in Example 1. The structural formulae of the carotenoids (capsanthin, capsorubin, cucurbitaxanthin A, other derivatives, etc.) identified as a result of ¹H NMR analysis are shown in the following formulae.

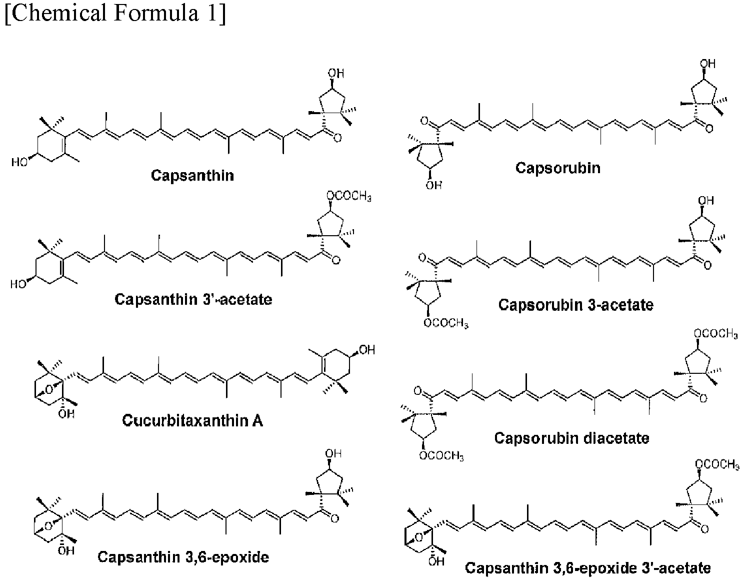

### SEQUENCE LISTING FREE TEXT

SEQ ID NO: 3 is obtained by substituting the 34th to 36th positions of the base sequence encoding S-Tag with the sequence encoding phenylalanine.
SEQ ID NO: 4 is one in which the 12th position in the amino acid sequence of S-Tag is substituted with phenylalanine.
SEQ ID NOs: 5 and 6 represent the base sequence and amino acid sequence of CaCCS into which S-Tag has been introduced, respectively.
SEQ ID NOs: 7 and 8 represent the base sequence and amino acid sequence of CaCCS into which S-Tag has been introduced via a linker, respectively.
SEQ ID NOs: 9 and 10 represent the base sequence and amino acid sequence of CaZEP into which S-Tag has been introduced, respectively.
SEQ ID NOs: 11 and 12 represent the base sequence and amino acid sequence of CaZEP into which S-Tag has been introduced via a linker, respectively.

## Claims

1. A transformant in which
a first promoter, and an upstream gene of a carotenoid biosynthesis gene including a crtY and crtZ operably linked to the first promoter, and
a second promoter having higher promoter intensity than that of the first promoter, and a ZEP gene and a CCS gene operably linked to the second promoter
have been introduced into a host cell.

2. The transformant according to claim 1, wherein a difference in promoter intensity between the first promoter and the second promoter is equal to or greater than a difference in promoter intensity between the promoter unique to the carotenoid biosynthesis gene and PBAD.

3. The transformant according to claim 1 or 2, wherein the upstream gene further comprises crtI; crtI and crtB; or crtI, crtB and crtE.

4. The transformant according to any one of claims 1 to 3, wherein the first promoter is Plac or a promoter unique to a carotenoid biosynthesis gene.

5. The transformant according to any one of claims 1 to 4, wherein the second promoter is PBAD or Ptac.

6. The transformant according to any one of claims 1 to 5, wherein
additionally, an S protein gene, an S tag gene, and a linker gene have been introduced into the host cell,
the S-tag gene is linked to each of the ZEP gene and the CCS gene, and
the linker gene is interposed and linked between the ZEP gene and the S-tag gene and/or between the CCS gene and the S-tag gene.

7. The transformant according to claim 6, wherein the S-tag encoded by the S-tag gene is any one of the following polypeptides (1) and (2):
(1) a polypeptide comprising a base sequence encoding the amino acid sequence set forth in SEQ ID NO: 4;
(2) a polypeptide that has a sequence identity of 90% or more other than the 12th sequence with respect to the amino acid sequence set forth in SEQ ID NO: 4 and specifically binds to S protein.

8. The transformant according to any one of claims 1 to 7, wherein the linker gene is interposed and linked at least between the ZEP gene and the S-tag gene.

9. The transformant according to any one of claims 1 to 8, wherein the linker gene is interposed and linked between the ZEP gene and the S-tag gene, and is not interposed between the CCS gene and the S-tag gene.

10. A production method for a carotenoid composition, comprising a step of culturing the transformant according to any one of claims 1 to 9.

11. The production method according to claim 10, wherein the carotenoid composition comprises capsanthin.

12. The production method according to claim 10 or 11, wherein the carotenoid composition comprises capsorubin, capsanthin 3'-acetate, capsorubin 3-acetate, cucurbitaxanthin A, capsorubin diacetate, capsanthin 3,6-epoxide, and/or capsanthin 3,6-epoxide 3'-acetate.
